Europäisches Patentamt

European Patent Office

Office européen des brevets

(11) Veröffentlichungsnummer: **0 162 426**
**A2**

(12) **EUROPÄISCHE PATENTANMELDUNG**

(21) Anmeldenummer: 85106116.8

(22) Anmeldetag: 18.05.85

(51) Int. Cl.⁴: **A 61 K 37/54**
**//C12N9/50**

(30) Priorität: 25.05.84 DE 3419581

(43) Veröffentlichungstag der Anmeldung:
27.11.85 Patentblatt 85/48

(84) Benannte Vertragsstaaten:
AT BE CH DE FR GB IT LI LU NL SE

(71) Anmelder: BEHRINGWERKE Aktiengesellschaft
Postfach 1140
D-3550 Marburg 1(DE)

(72) Erfinder: Falke, Jürgen, Dr.
Oberer Eichweg 22
D-3550 Marburg 1(DE)

(74) Vertreter: Meyer-Dulheuer, Karl-Hermann, Dr. et al,
HOECHST Aktiengesellschaft Zentrale Patentabteilung
Postfach 80 03 20
D-6230 Frankfurt/Main 80(DE)

(54) Verfahren zur Gewinnung einer Faktor XIII-Präparation sowie ihre Verwendung.

(57) Es wird ein Verfahren zur Gewinnung einer Faktor XIII-Präparation beschrieben, worin aus einem Plazentenextrakt der Faktor XIII mit einem Alkohol ausgefällt, an einen. Anionenaustauscher adsorbiert, der Austauscher gewaschen und der Faktor XIII eluiert wird.

Die gewonnene Faktor XIII-Präparation kann zur Behandlung von Blutgerinnungstörungen verwendet werden.

EP 0 162 426 A2

Verfahren zur Gewinnung einer Faktor XIII-Präparation
sowie ihre Verwendung

---

Die Erfindung betrifft ein Verfahren zur Gewinnung von
einer Faktor XIII-Präparation aus Plazenta. Diese Präparation kann zur Behandlung von Blutgerinnungsstörungen
verwendet werden.

In den deutschen Patentschriften 20 63 069 und 20 63 070
sind bereits Verfahren zur Herstellung eines Arzneimittels mit fibrinstabilisierender Wirkung, was gleichbedeutend mit faktor XIII-enthaltend ist, beschrieben. In
diesen beiden vielstufigen Verfahren wird der Faktor
XIII mit Diaminoethoxy-acridinlactat gefällt.
Die Verwendung dieses Fällungsmittels ist kostspielig.
Ein weiteres ebenfalls vielstufiges Verfahren ist aus
der europäischen Patentschrift 0011739 bekannt, worin in
einem Verfahrensschritt der Faktor XIII mit einem Alkylenoxid-Polymer gefällt wird.

Es wurde nun überraschenderweise gefunden, daß sich eine
Faktor XIII-Präparation aus Plazenten in höherer Ausbeute als nach den Verfahren des Standes der Technik gewinnen läßt, wobei von einer Ethanolfällung eines Salzextrakts aus Plazenten ausgegangen und die aufgelöste
Fällung an DEAE-Zellulose adsorbiert wird, Verunreinigungen durch Waschen des Austauschers weitgehend entfernt werden und der Faktor XIII eluiert wird.

Gegenstand der Erfindung ist deshalb ein Verfahren zur Gewinnung einer Faktor XIII-Präparation aus einem wässrigen Extrakt aus Plazenten, dadurch gekennzeichnet, daß der Faktor XIII mit einem niederen Alkanol gefällt, der Rückstand in einer wässrigen Lösung gelöst, die Lösung mit einem Anionenaustauscher in Kontakt gebracht, die Flüssigkeit abgetrennt, der Austauscher mit einer Faktor XIII nicht desorbierenden Flüssigkeit gewaschen und der Faktor XIII eluiert wird.

Für die Alkanol-Fällung wird vorzugsweise Ethanol verwendet, wobei die Konzentration des Alkohols auf 100 - 300 ml/l, vorzugsweise 150 - 300, eingestellt wird. Das pH beträgt 7 - 9, vorzugsweise 7,5 -7,7. Die Temperatur wird zwischen 0 und -10°C, vorzugsweise -5 und -7°C gehalten.

Der Rückstand wird in einer wässrigen Flüssigkeit bei einer Leitfähigkeit von weniger als 10 mS und einem pH von 5 - 9 gelöst, gegebenenfalls filtriert, und mit einem Anionenaustauscher, vorzugsweise DEAE-Zellulose, zusammengebracht, wobei die Menge an feuchter Austauschermasse 100 - 500, vorzugsweise 200 - 300 ml/l Flüssigkeit beträgt. Flüssigkeit und Ionenaustauscher können vermischt werden, wobei die Kontaktdauer 0,5 - 5, vorzugsweise 1 Stunde beträgt, oder die Flüssigkeit kann über eine Säule mit dem Austauscher geleitet werden. Der pH-Wert soll zwischen 7 und 9 liegen.

Flüssigkeit und Ionenaustauscher werden getrennt und der Ionenaustauscher mit einer wässrigen Flüssigkeit mit einer Leitfähigkeit von bis zu 3, vorzugsweise von 1-2 mS und bei einem pH von 7 - 9, vorzugsweise 7,5 - 7,7 gewaschen, vorzugsweise bis die Waschflüssigkeit eiweißfrei ist.

Der Austauscher wird mit einer wässrigen Flüssigkeit, die eine Leitfähigkeit über 2 mS aufweist, bevorzugt einer Salzlösung, die einen Komplexbildner enthält, bevorzugt einer mindestens 5 g Kochsalz pro 1 Wasser enthaltenden Lösung behandelt, wodurch der Faktor XIII desorbiert wird, und die Lösung gegebenenfalls konzentriert oder zur Trockene gebracht.

Die Ausbeute beträgt bis zu 50 % der in den Plazenten enthaltenen Aktivität.

Aus dem Überstand der Alkanol-Fällung können Albumin und aus der Anionenaustauscher-behandelten Lösung und aus den Waschflüssigkeiten des Ionenaustauschers Immunglobuline gewonnen werden.

Die erhaltene Faktor XIII-Präparation kann gegebenenfalls nach weiteren Maßnahmen zur Behandlung von Blutgerinnungsstörungen verwendet werden.

Beispiel

10,3 1 eines durch Behandeln von zerkleinertem Plazentenmaterial mit der gleichen Gewichtsmenge einer 5 g Kochsalz pro Liter enthaltenden wässrigen Lösung erhaltenen Extrakts wurden mit 5 mmol EDTA versetzt und bei pH 7,6 wurde bei -6°C soviel Ethanol zugegeben, daß die Mischung 250 ml Ethanol pro 1 enthielt. Man ließ eine Stunde rühren, fügte 50 g/l Celite J 2 Filterhilfsmittel (vorgewaschen mit 5 mmol/1 EDTA-Lösung) zu und filtrierte. Der Rückstand wurde dreimal in 1.6 1 einer je 5 mmol/1 Tris und EDTA enthaltenden Lösung aufgeschlämmt, gerührt und filtriert. Die vereinigten Eluate wurden über ein Ultrafilter auf 1.590 ml konzentriert, die 10.600 E Faktor XIII enthielten.

Das Konzentrat, das eine Leitfähigkeit von 2 mS besaß, wurde mit 375 g feuchter DEAE-Zellulose 2 Stunden lang gerührt. Die Eiweißlösung wurde über ein Filter abgesaugt und der Filterkuchen mit 1.350 ml eines je 5 mmol Tris und EDTA pro 1 enthaltenden Puffers gewaschen.

Zur Desorption wurde die DEAE-Zellulose in 1.600 ml einer 8,5 g NaCl und 5 mmol EDTA pro 1 enthaltenden wässrigen Lösung pH 7,2, 45 Minuten lang gerührt. Dies wurde nach Filtration der Lösung 2 mal wiederholt. Die Eluate wurden vereinigt. Sie enthielten 8.302 E Faktor-XIII-Aktivität. Vor Weiterverarbeitung kann diese Proteinlösung über Ultrafilter konzentriert oder lyophilisiert werden.

Patentansprüche:

1. Verfahren zur Gewinnung einer Faktor XIII-Präparation aus einem wässrigen Extrakt aus Plazenten, dadurch gekennzeichnet, daß der Faktor XIII mit einem niederen Alkanol gefällt, der Rückstand in einer wässrigen Lösung gelöst, die Lösung mit einem Anionenaustauscher in Kontakt gebracht, die Flüssigkeit abgetrennt, der Austauscher mit einer Faktor XIII nicht desorbierenden Flüssigkeit gewaschen und der Faktor XIII eluiert wird.

2. Verfahren nach Anspruch 1, dadurch gekennzeichnet, daß das Alkanol Ethanol ist und die Konzentration des Alkohols im Gemisch 150 - 300 ml/l ist.

3. Verfahren nach Anspruch 1, dadurch gekennzeichnet, daß der Anionenaustauscher DEAE-Zellulose ist.

4. Verfahren nach Anspruch 2, dadurch gekennzeichnet, daß der Ionenaustauscher mit einer wässrigen Flüssigkeit mit einer Leitfähigkeit von 1-2 mS gewaschen wird.